# EUROPÄISCHE PATENTSCHRIFT

(11) **EP 0 359 951 B1**
(45) Veröffentlichungstag und Bekanntmachung des Hinweises auf die Patenterteilung: **26.01.1994**
(21) Anmeldenummer: 89113933.9
(22) Anmeldetag: 28.07.1989
(51) Int. Cl.: A61K 35/78, A61K 31/35

(54) **Verwendung eines Ginkgo-Extraktes gegen Metastasen**
Use of a ginkgo extract against metastases
Utilisation d'un extrait de ginkgo contre les métastases

(30) Priorität: 21.09.1988 DE 3832056
(43) Veröffentlichungstag der Anmeldung: 28.03.1990
(73) Patentinhaber: Scholle, Helmut, Dr. med., D-38126 Braunschweig (DE)
(72) Erfinder: Scholle, Helmut, Dr. med., D-38126 Braunschweig (DE)
(74) Vertreter: Huber, Bernhard, Dipl.-Chem.

(56) Entgegenhaltungen:
- EP-A- 0 303 277
- EP-A- 0 352 146
- US-A- 4 751 224
- CHEM. PHARM. BULL., BAND 35, NR. 7, 1987, SEITEN 3016-3020; H. ITOKAWA ET AL. *SEITE 3016, ZUSAMMENFASSUNG; ABSCHNITT 1*
- PATENT ABSTRACTS OF JAPAN, BAND 13, NR. 233, (C-601) (3581), 29. MAI 1989; & JP-A-1 042 426 (DAICEL CHEM. IND. LTD) 14-02-1989, ZUSAMMENFASSUNG
- CHEM.PHARM.BULL., BNAND 37, NR. 6, JUNI 1989, SEITEN 1619-1621, PHARMAC. SOCIETY OF JAPAN; H. ITOKAWA ET AL. *SEITE 1619, SPALTE 1-SPALTE 2, ZEILE 3*
- JOURNAL DE PHARMACIE DE BELGIQUE, BAND 41, NR. 5, ERGäNZ 4, SEPT.-OKT. 1986, SEITEN 30-39; BE; T. SEVENET, *SEITE 37, SPALTE 1, ZEILEN 5-11*
- INT.J. IMMUNOPHARMAC. BAND 12, NR. 1, 1990, SEITEN 57-65, INTERNAT. SOCIETY FOR IMMUNOPHARMAC. GB; D.FECCHIO ET AL. *ZUSAMMENFASSUNG*

## Beschreibung

Die Erfindung betrifft die Verwendung eines Trockenextraktes aus Ginkgo-Biloba-Blättern.

Unter dem Warenzeichen "Tebonin" bringt die Firma Dr. Willmar Schwabe ein Naturstoffpräparat als Injektions- und Infusionslösung auf den Markt, und zwar gegen zerebrale und periphere Mangeldurchblutung und Mangelernährung. Mit diesem Medikament werden bei Hirnleistungsschwäche die Symptome Schwindel, Ohrensausen und vor allem gefäßbedingte Kopfschmerzen und Sehstörungen gebessert. Bei gefäßbedingter Innenohrschwerhörigkeit wirkt das Medikament auf das gestörte Hörvermögen und das Sprachverständnis. Gesteigert werden sollen Merk- und Konzentrationsfähigkeit und das intellektuelle Leistungsvermögen. Gebessert werden Angstzustände, depressive Verstimmungen und neurologische Störungen.

Dieses vorbekannte Medikament wird u. a. in Form von Infusionsampullen verwendet, wobei eine Infusionsampulle zu 25 ml enthält: Extracta Ginkgo bilobae e folibus siccum purissimum pro injektione 87,5 mg standardisiert auf 21,0 mg Ginkgoflavonglykoside.

Die Erfindung besteht in der Verwendung eines Trockenextraktes aus Ginkgo-Biloba-Blättern zur Behandlung metastatischer Krebserkrankungen. Außerdem besteht die vorliegende Erfindung in der Verwendung des Trockenextraktes aus Gingko-Biloba-Blättern als Infusion von 7, 17,5 oder 87,5 mg oder eines Vielfachen der Dosis des genannten Trockenextraktes, standartisiert auf 1,68, 4,2 und 21,0 mg oder einer vielfachen Dosis von Ginkgoflavonglykosiden im Extrakt.

Dabei ist es vorteilhaft, wenn das genannte Trockenextrakt als Vorabinfusion vor der Gabe zytostatischer Chemotherapeutika und und ggf. als Begleittherapie oral bzw. parenteral verabreicht wird.

Es hat sich gezeigt, daß Chemotherapeutika in Verbindung mit Ginkgoflavinglykosiden einen größeren krebswachstumshemmenden Effekt zeigen als die kombinierte Anwendung verschiedener Zytostatika allein. Die Dosierung der zytostatischen Chemotherapie, sowohl bei Mono- als auch bei kombinierter Chemotherapie, läßt sich somit unter Anwendung der Ginkgo-Extrakte reduzieren. Noch höhere Wirksamkeit bzw. Potenzierung des krebswachstumhemmenden Effektes läßt sich durch mehrfache Vorabinfusionen von Ginkgo-Extrakt bei gesplitterter kleiner Chemotherapeutika-Dosierung erzielen.

Daraus läßt sich das Ergebnis herleiten, wonach Ginkgo-Extrakte einen potenzierenden Effekt in der krebswachstumshemmenden Wirkung verschiedener Zytostatikamittel hervorrufen.

Hauptproblem der Tumortherapie mit Zytostatika ist die fehlende Selektivität für das Tumorgewebe. Die maximal mögliche Dosierung wird daher unabhängig vom therapeutischen Effekt durch die schädigende Wirkung auf andere Organe - speziell das blutbildende Organ - limitiert.

Ginkgo biloba-Extrakt (GBE) ist ein Extrakt aus getrockneten Blättern des Ginkgo biloba-Baumes. Er enthält u.a. Terpene wie wie Ginkgolide, Bilobalide, Flavonheteroside und Proanthocyanidine. GBE entfaltet eine Wirkung auf Hämodynamik, Rheologie und energetische Zellstoffwechsel.

Ginkgo biloba-Extrakt besitzt die Eigenschaft, sogenannte freie Sauerstoffradikale einzufangen. Da die zellzerstörende Wirkung der Chemotherapie mit einer gesteigerten Produktion von freien Radikalen einhergeht, könnte die Eigenschaft, freie Radikale einzufangen, speziell in den besser perfundierten Stromgebieten zumindest eine Verminderung von unerwünschten Wirkungen der Chemotherapie erklären.

Ginkgo biloba-Extrakt kann Wirkungen des plättchenaktivierenden Faktors (PAF) antagonisieren. PAF ist eine körpereigene Substanz, die in einer Vielzahl von physiologischen Vorgängen eine entscheidende Rolle zu spielen scheint. Neben der blutplättchenaktivierenden Funktion (daher der Name) spielt PAF eine entscheidende Rolle als Entzündungsmediator. Ein Einfluß von PAF auf das Immunsystem gilt als gesichert, ist aber noch nicht vollständig untersucht.

### Beispiel

GBE liegt z.B. zur parenteralen Applikation als Lyophilisat in Ampullen a 50 mg Wirksubstanz vor. Mitgeliefert werden Ampullen mit jeweils 3 ml Lösungsmittel. Auch Fertiglösungen des Extraktes können in Anwendung gebracht werden (z.B. das vorstehend erwähnte "Tebonin").

Für eine Applikation beträgt die Tagesdosis 200 mg GBE entsprechend 4 Ampullen oder ein Vielfaches hiervon. Dazu wird das Lyophilisat der 4 Wirkstoffampullen in dem beiliegenden Lösungsmittel aufgelöst (jede Wirkstoffampulle in jeweils 3 ml Lösungsmittel) und in 250 ml isotonischer Kochsalzlösung über 30 Minuten intravenös infundiert.

Behandelt wurden Patienten mit Lungenmetastasen und Lebermetastasen, die bereits zwei zytostatische Behandlungszyklen nach tumorabhängiger Standardtherapie appliziert bekommen haben. Bei zum Beispiel Mamma-Carcinom-Patienten hat sich der additiv bzw. potenzierende Effekt zu einem Epirubincin-Cyclophosphamid Schema bewährt; bei colorektalen Tumoren in Kombination von Ginkgo-biloba-Extrakt mit Leukoverin + Fluorouracil.

Bei Kombinationen anderer Chemotherapieprotokolle ist der gleiche Effekt zu erwarten.

Vor dem dritten Behandlungszyklus muß der Befund durch Computertomographie (Lebermetastasen) oder Röntgen (Lungenmetastasen) gesichert werden. Zusätzlich erfolgt die Kontrolle des Blutbildes und der Laborparameter, die im Rahmen einer chemotherapeutischen Tumorbehandlung erforderlich sind und die Verlaufsbeurteilung der entsprechend zu behandelnden Tumorpatienten möglich macht.

Unmittelbar vor jeder parenteralen Gabe des Chemotherapeutikums im dritten Behandlungszyklus erhalten die Patienten eine intravenöse Infusion von 200 mg GBE.

Die Kontrolluntersuchung soll zu dem Zeitpunkt erfolgen, zu dem der größte therapeutische Effekt zu erwarten ist. Bei dieser Kontrolluntersuchung kommen dieselben Verfahren zur Anwendung wie bei der Untersuchung vor dem dritten Behandlungszyklus.

In Verbindung mit GBE erscheinen anthracycline und alkylierende Substanzen als zytostatische Chemotherapeutika besonders wirksam.

## Patentansprüche

1. Verwendung eines Trockenextraktes aus Ginkgo-biloba-Blättern zur Herstellung eines Arzneimittels für eine therapeutische Anwendung bei metastatischen Krebserkrankungen in Kombination mit Chemotherapeutika.

2. Verwendung nach Anspruch 1 als Vorabinfusion vor der Gabe zytostatischer Chemotherapeutika.

3. Verwendung nach Anspruch 1 oder 2 als Infusion von 7, 17,5 oder 87,5 mg oder eines Vielfachen der Dosis des genannten Trockenextraktes standardisiert auf 1,68, 4,2 und 21,0 mg oder einer vielfachen Dosis von Ginkgoflavonglvkosiden im Extrakt.

4. Verwendung nach Anspruch 1 oder 2 als intravenöse Infusion von Gingko biloba-Extrakt (GBE) als Lyophilisat aufgelöst in einem Lösungsmittel und vermischt mit isotonischer Kochsalzlösung.

5. Verwendung nach Anspruch 4, gekennzeichnet durch eine Tagesdosis von 200 mg GBE, aufgelöst in 12 ml Lösungsmittel und vermischt mit 250 ml isotonischer Kochsalzlösung.

## Claims

1. Use of a dry extract of Ginkgo biloba leaves for the preparation of a medicament for a therapeutic application in metastatic cancerous diseases in combination with chemotherapeutic agents.

2. Use according to Claim 1 as a preinfusion before the dose of cytostatic chemotherapeutic agents.

3. Use according to Claim 1 or 2 as an infusion of 7, 17.5 or 87.5 mg or a multiple of the dose of said dry extract standardised to 1.68, 4.2 or 21.0 mg or a multiple dose of Ginkgo flavone glycosides extract.

4. Use according to Claim 1 or 2 as an intravenous infusion of Ginkgo biloba extract (GBE) dissolved as lyophilisate in a solvent and mixed with isotonic sodium chloride solution.

5. Use according to Claim 4, characterised by a daily dose of 200 mg GBE, dissolved in 12 ml of solvent and mixed with 250 ml of isotonic sodium chloride solution.

## Revendications

1. Utilisation d'un extrait sec de feuilles de ginkgo bilobé pour la préparation d'un médicament pour une utilisation thérapeutique dans les maladies cancéreuses métastatiques, en combinaison avec des agents chimiothérapeutiques.

2. Utilisation selon la revendication 1, sous forme de perfusion préalable avant l'administration des agents chimiothérapeutiques cytostatiques.

3. Utilisation selon la revendication 1 ou 2, sous forme de perfusion de 7, 17,5 ou 87,5 mg ou d'un multiple de la dose dudit extrait sec normalisé à 1,68, 4,2 ou 21,0 mg ou un multiple de la dose de flavonosides de ginkgo dans l'extrait.

4. Utilisation selon la revendication 1 ou 2 en perfusion intraveineuse d'extrait de ginkgo bilobé (EGB) sous forme d'un lyophilisat dissous dans un solvant et mélangé avec une solution isotonique de chlorure de sodium.

5. Utilisation selon la revendication 4, caractérisée par une dose journalière de 200 mg d'EGB dissous dans 12 ml de solvant et mélangé avec 250 ml de solution isotonique de chlorure de sodium.
